# FASCICULE DE BREVET EUROPEEN

(11) **EP 1 971 858 B1**
(45) Date de publication et mention de la délivrance du brevet: **24.08.2016**
(21) Numéro de dépôt: 06847094.7
(22) Date de dépôt: 20.12.2006
(51) Int. Cl.: G01N 33/08, A01K 43/00

(54) **DISPOSITIF DE MIRAGE D'OEUFS**
VORRICHTUNG ZUM DURCHLEUCHTEN VON EIERN
EGG EXAMINING DEVICE

(30) Priorité: 21.12.2005 FR 0512998
(43) Date de publication de la demande: 24.09.2008
(73) Titulaire: CEVA SANTE ANIMALE, 33501 Libourne Cedex (FR)
(72) Inventeur: NADREAU, Michael, F-29200 Brest (FR); CROGUENNEC, Robert, F-29290 Milizac (FR)
(74) Mandataire: Vidon Brevets & Stratégie
(86) Numéro de dépôt international: PCT/FR2006/002815
(87) Numéro de publication internationale: WO 2007/077337

(56) Documents cités:
- WO-A-99/14589
- WO-A-2004/023136
- US-A- 4 161 366
- US-A1- 2002 075 476
- PATENT ABSTRACTS OF JAPAN vol. 1995, no. 02, 31 mars 1995 (1995-03-31) & JP 06 308098 A (OTAX KK), 4 novembre 1994 (1994-11-04)

## Description

La présente invention concerne un dispositif de mirage d'oeufs pour différencier les oeufs fécondés des oeufs non-fécondés.

Dans l'industrie de la volaille, en particulier l'industrie du poulet, il est connu des dispositifs de mirage automatique d'oeufs utilisant la transparence de l'oeuf pour différencier les oeufs fécondés et non-fécondés. Ces dispositifs comprennent des moyens d'émission pour émettre un flux lumineux en direction d'un oeuf à mirer, des moyens de réception pour recevoir le flux lumineux passant à travers l'oeuf, et des moyens de traitement informatique du flux lumineux reçu par lesdits moyens de réception pour déterminer l'état de l'oeuf. En fonction du taux d'adsorption du faisceau lumineux passant au travers de l'oeuf, ou taux de transparence de l'oeuf, les moyens de traitement informatique permettent de différencier les oeufs fécondés, à savoir les oeufs comprenant un embryon, des oeufs non-fécondés incluant les oeufs clairs et les oeufs pourris. Certains dispositifs permettent en outre de différencier les oeufs fécondés vivants, comprenant un embryon vivant, des oeufs fécondés non-vivants comprenant un embryon mort.

De tels dispositifs de mirage sont par exemple décrit dans US 2002/075476, WO 99/14589 ou WO 2004/023136.

Ces dispositifs de mirage comportent classiquement un convoyeur d'acheminement destiné à transporter les oeufs placés dans leurs casiers ou plateaux d'incubation horizontaux, les moyens d'émission et les moyens de réception étant disposés de part et d'autre du convoyeur d'acheminement. Les moyens d'émission sont généralement constitués d'émetteurs formés de sources lumineuses de type lampe à filament ou diode électroluminescente, classiquement d'environ 12 W, les moyens de réception étant formés de récepteurs de type photodiode ayant des surfaces de réception de l'ordre de 0,5 mm². Pour obtenir des mesures de transparence satisfaisantes, les émetteurs et les récepteurs sont disposés en vis-à-vis selon un même plan vertical.

Le principal défaut de ces systèmes de mirage vertical est leur faible immunité aux déchets provenant des plateaux d'oeufs. Les déchets, tels que des morceaux de coquille ou contenu d'oeufs cassés, plumes ou matières organiques diverses, tombent par gravité sur les émetteurs ou récepteurs placés en-dessous des plateaux, ce qui entraîne des variations et des erreurs de mesures. Pour limiter ces variations et erreurs de mesure, un entretien très régulier du dispositif de mirage est donc nécessaire. A cet effet, il a été proposé, notamment dans le document brevet FR 2 768 517 de placer un écran de protection entre les plateaux et les émetteurs placés en dessous des plateaux. Les flux lumineux des émetteurs passent à travers l'écran de protection, et des moyens de nettoyage automatique sont associés audit écran de protection. De tels systèmes mécaniques se révèlent encombrants et de conception complexe.

US 4 161 366 A décrit un dispositif selon le préambule de la revendication 1. Le but de la présente invention est de proposer un dispositif de mirage palliant les inconvénients précités.

A cet effet, la présente invention a pour objet un dispositif de mirage automatique d'oeufs permettant de différencier les oeufs fécondés des oeufs non-fécondés, et éventuellement de différencier les oeufs fécondés vivants des oeufs fécondés non-vivants, comprenant des moyens d'émission d'un flux lumineux en direction d'un oeuf à mirer, des moyens de réception du flux lumineux passant à travers l'oeuf, et des moyens de traitement informatique du flux lumineux reçu par lesdits moyens de réception pour déterminer l'état fécondé ou non-fécondé de l'oeuf, caractérisé en ce que lesdits moyens d'émission comprennent pour chaque oeuf à mirer au moins une source laser cohérente formant un faisceau optique cohérent en direction de l'oeuf.

L'utilisation d'une source laser cohérente Selon l'invention permet d'obtenir un faisceau optique cohérent et fin qui est plus concentré et qui pénètre mieux la coquille de l'oeuf que le flux lumineux d'une simple diode électroluminescente, ce qui permet à la source lumineuse de ne pas être nécessairement disposée du côté opposé ou juste en face des moyens de réception. L'utilisation d'une source laser cohérente permet ainsi un multi-positionnement de la source lumineuse et des moyens de réception. Selon l'invention, il est donc possible de disposer la source lumineuse de sorte qu'elle soit protégée des déchets qui tombent des plateaux d'oeufs. Le dispositif selon l'invention nécessite un entretien moins régulier et une meilleure stabilité et meilleure validité des résultats.

Selon un mode de réalisation, ladite source laser est disposée en-dessous des oeufs à mirer, le faisceau optique formant un angle non nul par rapport à la verticale, par exemple compris entre 5 et 45°, par exemple de l'ordre de 15°. Avantageusement, ladite source laser est disposée en-dessous d'une plaque protectrice. Grâce à son faisceau fin et cohérent, la source laser peut être disposée à une distance de l'oeuf plus importante que les sources lumineuses de l'art antérieur, sans illuminer les oeufs voisins du plateau, avec un angle de faisceau non nul par rapport à la verticale, de sorte que la source laser puisse être protégée des déchets, notamment sous une plaque protectrice.

Selon un autre mode de réalisation, ladite source laser est disposée au-dessus des oeufs à mirer, le faisceau cohérent formant un angle non nul par rapport à la verticale, les mesures étant avantageusement réalisée lorsque ledit faisceau optique atteint la moitié inférieure de l'oeuf.

Avantageusement, les moyens de réception sont disposés du côté du plan médian vertical de l'oeuf opposé à ladite source laser, de préférence au-dessus des oeufs à mirer.

Pour obtenir un faisceau optique plus fin, ladite source laser est avantageusement équipée de moyens de focalisation pour former un faisceau optique cohérent concentré. Selon un mode de réalisation, ladite source laser est constituée d'une diode laser, équipée de préférence d'un collimateur laser formant lesdits moyens de focalisation. Ladite source laser est avantageusement une source laser infrarouge ou une source laser rouge, de préférence une source laser infrarouge.

Selon un mode de réalisation lesdits moyens de réception comprennent pour chaque oeufs au moins une photodiode, de préférence infrarouge, ayant une surface de réception d'au moins 1 mm², de préférence équipée de moyens de focalisation du flux lumineux transmis à travers l'oeuf.

Selon un mode de réalisation, le dispositif de mirage selon l'invention comprend des moyens de convoyage pour transporter, de préférence en continu, des plateaux munis d'alvéoles dans lesquelles des oeufs à mirer sont disposés, lesdits moyens d'émission comprenant au moins une rangée de sources laser cohérente disposées transversalement à la direction d'avancement des plateaux pour émettre au moins un faisceau optique vers chacun des oeufs d'une même rangée d'un plateau, et au moins une rangée de moyens de réception pour recevoir les flux lumineux provenant des oeufs d'une rangée d'un plateau.

Le dispositif de mirage comprend en outre des moyens de mesure pour mesurer la distance de l'oeuf à mirer par rapport aux moyens de réception, le traitement informatique du flux lumineux reçu par lesdits moyens de réception étant effectué en fonction de la distance mesurée pour diminuer les variations de rayonnement provoquées par les différentes hauteurs d'oeufs.

Selon une particularité, le dispositif de mirage comprend en outre des moyens de synchronisation, tels qu'un récepteur supplémentaire pour la réception d'ondes lumineuses ambiantes et un dispositif de type horloge, pour synchroniser les mesures de transparence effectuées par les moyens de réception et les impulsions de la source laser sur la fréquence de l'énergie des ondes lumineuses ambiantes, au voisinage du sommet S des périodes T de l'énergie des ondes lumineuses ambiantes. Cette synchronisation selon l'invention constitue une solution simple et peu coûteuse d'un point de vue mécanique, électronique et optique pour s'immuniser contre les ondes lumineuses ambiantes. Chaque mesure de transparence comprend l'acquisition au voisinage d'un sommet S d'une première valeur val1 correspondant au niveau d'énergie lumineuse sur les moyens de réception sans que sa source laser associée soit activée, puis d'une deuxième valeur val2 correspondant au niveau d'énergie lumineuse sur le récepteur avec sa source laser associée activée, la mesure de transparence étant obtenue par simple soustraction de la première valeur val1 à la deuxième valeur val2.

L'invention sera mieux comprise, et d'autres buts, détails, caractéristiques et avantages apparaîtront plus clairement au cours de la description explicative détaillée qui va suivre de deux modes de réalisation particuliers actuellement préférés de l'invention, en référence au dessin schématique annexé. Sur ce dessin :
- la figure 1 représente une vue partielle en coupe longitudinale d'un dispositif de mirage selon un premier mode de réalisation de l'invention ;
- la figure 2 représente une vue en coupe transversal selon le plan II-II de la figure 1 ;
- la figure 3 représente une vue schématique de côté d'un émetteur et d'un récepteur selon une variante du premier mode de réalisation ;
- la figure 4 représente une vue schématique de côté d'un émetteur et d'un récepteur d'un dispositif de mirage selon un deuxième mode de réalisation de l'invention ;
- la figure 5 représente un graphe illustrant l'énergie lumineuse provenant de sources lumineuses ambiantes en fonction du temps, avant une opération de mirage ;
- la figure 6 représente un graphe illustrant l'énergie lumineuse provenant d'une source laser et de sources lumineuse ambiantes en fonction du temps lors d'une opération de mirage ; et,
- les figures 7 et 8 représentent des dispositions différentes de sources laser sur une rampe.

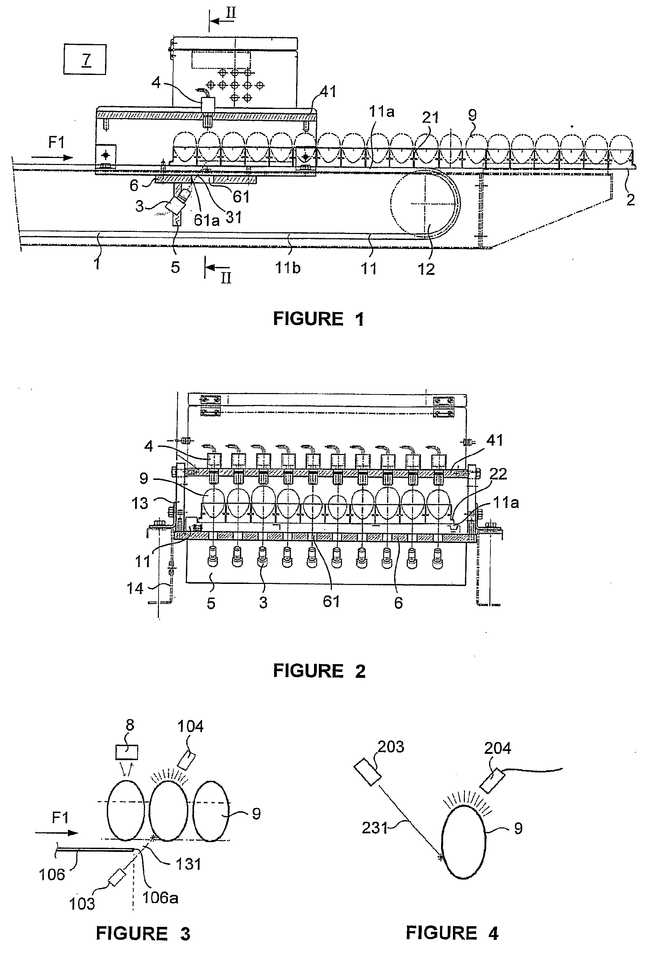

En référence aux figures 1 et 2, le dispositif selon l'invention comprend des moyens de convoyage 1 pour transporter des plateaux d'incubation d'oeufs 2, suivant un chemin de convoyage le long duquel sont disposés des moyens d'émission ou émetteurs 3, et des moyens de réception ou récepteurs 4.

Les moyens de convoyage 1 comprennent un convoyeur de type à bande sans fin, déplaçant les plateaux dans la direction d'avancement F1. Le convoyeur est formé de deux courroies ou de deux chaînes parallèles 11 à déplacement synchrone, chacune montée en boucle sur une roue de renvoi amont et une roue de renvoi aval 12. L'espacement entre les deux chaînes est déterminé de sorte que les plateaux 2 reposent par leurs bords latéraux 22 sur les brins supérieurs 11a des deux chaînes.

Dans l'exemple illustré, le dispositif est configuré pour le traitement d'oeufs 9 disposés sur des plateaux 2 de type « rectangulaire », connus en soi, comprenant des rangées transversales parallèles de logements ou alvéoles 21 à fond ouvert, tels que les plateaux d'incubation vendus sous la dénomination commerciale « Petersime », les alvéoles des rangées étant alignées longitudinalement.

Les émetteurs et récepteurs sont adaptés pour effectuer le traitement des oeufs d'une même rangée. Le dispositif comprend pour chaque oeufs à mirer, un émetteur 3 formé d'une source laser cohérente, de préférence infrarouge, et un récepteur infrarouge 4 pour la réception optique du flux lumineux passant au travers de l'oeuf.

Les sources laser sont disposées côte à côte sur une rampe 5 formée d'une plaque verticale placée transversalement à la direction d'avancement F1 sous le brin supérieur 11a du convoyeur, par exemple entre le brin inférieur 11b et le brin supérieur 11a. Les sources laser, au nombre de 10, sont assemblées sur la rampe de manière à être alignées avec les 10 alvéoles des rangées des plateaux défilant en continu au-dessus de la rampe, chaque source étant apte à émettre un faisceau lumineux 31, formant un angle non nul par rapport à la verticale, qui vient illuminer un côté latéral inférieur de chaque oeufs à mirer pour la prise de la mesure. La rampe est disposée sous une plaque protectrice horizontale 6 fixée sous le brin supérieur et munie d'une rangée d'ouvertures 61 pour le passage des faisceaux. Dans l'exemple illustré, la rampe est fixée à la plaque protectrice 6, en amont des ouvertures 61 par rapport à la direction d'avancement F1, ladite plaque étant fixée à une structure porteuse 13 elle-même assemblée au châssis 14 du convoyeur. Les sources sont décalées longitudinalement par rapport à l'extrémité amont 61a des ouvertures, et sont ainsi protégées par la plaque des éventuels déchets tombant des oeufs et/ou des plateaux. Le faisceau cohérent incliné 31 de chaque source laser est orienté pour venir illuminer le côté aval inférieur des oeufs à mirer, en passant au raz de l'extrémité amont 61a d'une ouverture 61. En variante, les ouvertures sont remplacées par une seule et unique fente transversale.

Les récepteurs 4 sont montés au-dessus du chemin de convoyage, côte à côte sur une rampe horizontale 41 placée transversalement à la direction d'avancement. Dans le présent exemple, la rampe 41 est formée d'une plaque montée horizontalement au-dessus des plateaux sur la structure porteuse 13 précitée. La rampe porte une rangée transversale de 10 récepteurs, chaque récepteur étant disposée sensiblement verticalement au-dessus d'une ouverture 61 de sorte que le récepteur soit sensiblement centré selon le plan médian vertical d'un oeuf, lorsque le point d'impact du faisceau optique 31 de la source laser associée est sensiblement au niveau de l'extrémité inférieure de l'oeuf.

Les récepteurs sont connectés à une unité de contrôle et de traitement informatique, représentée schématiquement sous la référence 7, qui détermine, en fonction de l'intensité du flux lumineux détecté par le récepteur, si un oeuf est fécondé ou non. Avantageusement, le traitement informatique permettra de différencier les oeufs fécondés vivants des oeufs non-fécondés et des oeufs fécondés non-vivant. Le dispositif de mirage selon l'invention peut être utilisé pour effectuer des opérations de mirage d'oeufs à 18 jours, à savoir normalement fécondés depuis 18 jours, éventuellement d'oeufs de 6 à 10 jours, et sera avantageusement placé en amont d'un dispositif d'enlèvement des oeufs non-fécondés et des oeufs fécondés non-vivants des plateaux et/ou d'un dispositif d'injection des oeufs fécondés vivants des plateaux.

Chaque source laser 3 est avantageusement constituée d'une diode laser infrarouge équipée d'un collimateur laser afin de concentrer davantage le faisceau lumineux et générer un faisceau optique très fin. Le faisceau cohérent de la source laser pénétrant mieux l'oeuf que les sources lumineuses de l'art antérieur, notamment qu'une simple diode électroluminescente, le dispositif est avantageusement équipée de sources laser de faible puissance par rapport aux sources lumineuses de l'art antérieur. A titre d'exemple, le dispositif comprend des diodes laser de 50 à 200 mW, par exemple de 150 mW.

Chaque récepteur 4 est constitué d'une photodiode de grande sensibilité, ayant une surface de réception d'au moins 1 mm², de préférence comprise entre 2 et 15 mm², mieux encore entre 5 et 8 mm², par exemple de l'ordre de 7 mm². Les photodiodes permettent une grande linéarité de mesure et donc une grande précision de mesure. Avantageusement, chaque photodiode est équipée d'une lentille de focalisation permettant de focaliser le flux lumineux pour ne pas mesurer de mauvaise information, comme les reflets par exemple.

Dans la variante de réalisation illustrée à la figure 3, le récepteur 104 n'est pas centré par rapport à l'oeuf mesuré. Chaque récepteur est incliné par rapport à la verticale et est décalé longitudinalement, du côté du plan médian vertical de l'oeuf qui est opposé à la source laser associée 103. Dans cette variante, les sources laser 103 sont disposées en-dessous d'une plaque 106 dépourvue d'ouvertures, les sources laser étant simplement décalées longitudinalement par rapport au bord transversal 106a de la plaque, le faisceau cohérent 131 passant à proximité dudit bord transversal.

L'utilisation d'une source laser cohérente permet d'éviter d'éclabousser les oeufs adjacents et le plateau qui entourent l'oeuf mesuré avec le rayonnement infrarouge, et ainsi d'éviter les reflets sur les oeufs et le plateau qui dégraderaient la mesure effectuée. Tel qu'illustré sur la figure 2, des variations importantes de taille et de forme peuvent apparaître entre les oeufs. Le faisceau fin de la source laser permet de minimiser les variations de distance entre l'émetteur et l'oeuf et donc d'assurer une meilleure stabilité des mesures de transparence aux variations de taille et de forme des oeufs.

Ces variations de taille des oeufs entraînent également des variations de distance entre le récepteur et l'oeuf. Afin de minimiser les variations de mesure dues à ces variations de distance, le dispositif comprend en outre, en référence à la figure 3, des capteurs de distance 8, par exemple de type émetteur/récepteur, disposés au-dessus des oeufs à mirer et connectés à l'unité contrôle et de traitement informatique. Chaque capteur mesure la distance le séparant du sommet de l'oeuf à mirer. L'unité de contrôle et de traitement informatique 7 applique alors en fonction de la distance mesurée un coefficient d'amplification sur le signal, transmis par le récepteur, représentatif de l'intensité du flux lumineux traversant l'oeuf. Le dispositif comprend par exemple une rampe transversale de 10 capteurs de distance, disposés au-dessus des plateaux, en amont des récepteurs, pour mesurer les distances des oeufs d'une même rangée.

La figure 4 illustre de manière schématique un deuxième mode de réalisation dans lequel les récepteurs 204 et les émetteurs 203, formés de sources laser cohérentes sont disposés au-dessus des plateaux d'oeufs. Le faisceau optique de chaque émetteur 203 est incliné par rapport à la verticale, de sorte que, pour effectuer la mesure, les points d'impact du faisceau cohérent 231 sur la coquille de l'oeuf à mirer lors du déplacement de ce dernier soient situés dans la moitié inférieure de l'oeuf. Les récepteurs sont disposés de manière analogue à ceux de la figure 3, du côté du plan médian vertical des oeufs opposé aux émetteurs. L'angle entre le faisceau cohérent et la surface de la coquille au point d'impact étant plus faible, le coefficient de pénétration du faisceau est moins important que dans le premier mode de réalisation illustré aux figures 1 et 3. La ligne de points d'impact pour la prise de la mesure de transparence sera située aussi bas que possible pour éviter les effets de reflet sur les oeufs adjacents et le plateau, tout en maintenant un coefficient de pénétration suffisant pour permettre la mesure par transparence.

Dans les exemples décrits précédemment, l'analyse d'un oeuf est effectuée au moyen d'un récepteur et d'un émetteur. Dans des variantes de réalisation, plusieurs récepteurs et/ou émetteurs peuvent être prévus pour l'analyse d'un même oeuf. Le dispositif peut en outre être configuré pour le traitement de plateaux d'incubation de type décalé, connus en soi, dans lesquels les rangées sont disposées en quinconce, tels que ceux vendus sous la dénomination commerciale « Chickmaster ». Les sources laser, ainsi que les récepteurs, seront alors disposés en quinconce sur leur rampe respective selon deux rangées transversales parallèles.

Pour réduire ou supprimer les erreurs de mesure de transparence dues aux ondes lumineuses ambiantes parasites des sources ambiantes artificielles, les mesures de transparence effectuées par les récepteurs et les impulsions des sources laser sont synchronisées sur la fréquence de l'énergie des ondes lumineuses ambiantes.

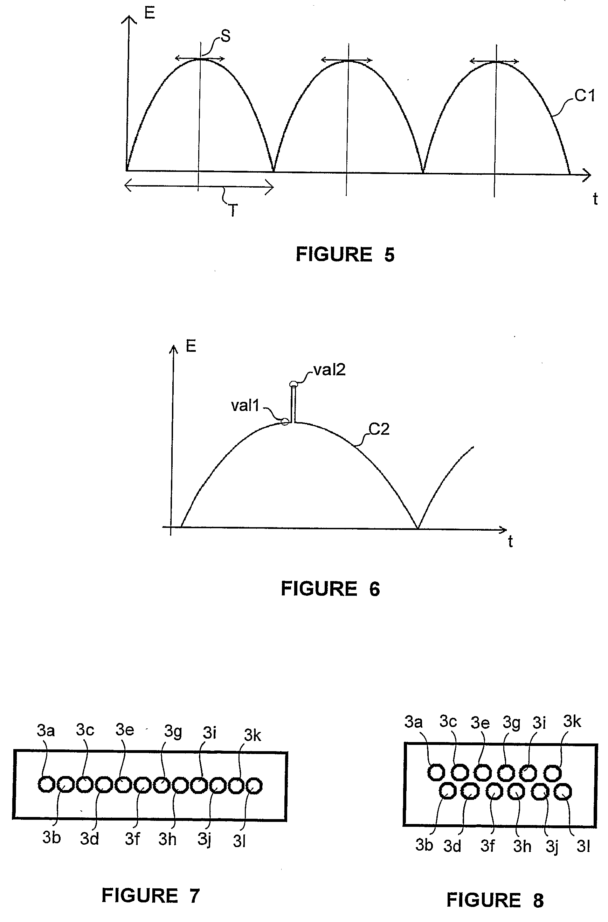

Avant toute opération de mirage, on effectue une mesure de l'énergie des ondes lumineuses ambiantes. La courbe C1 de la figure 5 représente l'énergie lumineuse E en fonction du temps t de ces ondes lumineuses ambiantes. Cette mesure est réalisée par un récepteur supplémentaire du dispositif de mirage et est transmise à l'unité de contrôle et de traitement informatique 7 pour synchroniser, au moyen d'un dispositif de type horloge par exemple, les mesures de transparence et les impulsions au voisinage du sommet S des périodes T de l'énergie des ondes lumineuses ambiantes, là où le coefficient de variation de l'énergie lumineuse, dit coefficient directeur, est le plus faible.

La courbe C2 de la figure 6 représente l'énergie lumineuse E en fonction du temps t des ondes lumineuses ambiantes et d'une source laser lors d'une mesure de transparence. Pour chaque mesure de transparence d'un oeufs par une paire source laser/récepteur, l'unité de contrôle commande le récepteur pour l'acquisition au voisinage du sommet S d'une première valeur Val1 correspondant au niveau d'énergie lumineuse sur le récepteur sans que sa source laser associée soit activée, puis d'une deuxième valeur Val2 correspondant au niveau d'énergie lumineuse sur le récepteur avec sa source laser associée activée. La mesure de transparence non parasitée par les ondes lumineuses ambiantes est alors obtenue par soustraction de la première valeur val1 à la deuxième valeur val2. Pour que le temps d'acquisition des deux valeurs soit le plus faible possible, et ainsi garantir une acquisition des deux valeurs au voisinage du sommet, l'ordre d'émission de la source laser est avantageusement réalisée par le récepteur associé.

Pour chaque oeufs à mirer, on effectue plusieurs mesures de transparences successives, par exemple au nombre de dix, synchronisées au voisinage de sommets S successifs.

Des mesures de transparence des oeufs d'une même rangée de plateau peuvent être effectuées au niveau du sommet d'une même période de l'énergie des sources lumineuses ambiantes. A titre d'exemple, dans le cas de dix mesures de transparence par oeuf, les mesures de transparence des oeufs sont effectuées sur les dix mêmes périodes T.

Pour éviter une perturbation entre les récepteurs des différentes paires sources laser/récepteur par superposition des énergies lumineuses des sources laser, le dispositif de mirage applique un ordre d'autorisation de cycle de mesure de transparence différent pour chaque paire émetteur/récepteur, de sorte qu'une seule source laser émette à la fois. A titre d'exemple, dans le cas d'une rampe de 12 sources laser référencées de 3a à 31, et disposées selon une rangée, tel qu'illustré à la figure 7, ou disposées en quinconce selon deux rangées tel qu'illustré à la figure 8, l'ordre d'émission des sources laser est le suivant : a, g, b, h, c, i, d, j, e, k, f, l. Les 12 sources laser peuvent émettre sur une même période de l'énergie des ondes lumineuses ambiantes, les premières à émettre émettant alors légèrement en amont de son sommet S, et les dernières émettant légèrement après son sommet S.

## Revendications

1. Dispositif de mirage automatique d'oeufs permettant de différencier les oeufs fécondés des oeufs non-fécondés, comprenant des moyens d'émission d'un flux lumineux en direction d'un oeufs a mirer, des moyens de réception du flux lumineux passant a travers l'oeuf, et des moyens de traitement informatique du flux lumineux reçu par lesdits moyens de réception pour déterminer l'état fécondé ou non-fécondé de l'oeuf, lesdits moyens d'émission (3, 103, 203) comprenant pour chaque oeufs à mirer au moins une source laser cohérente formant un faisceau optique cohérent (31, 131, 231) en direction de l'oeuf (9),
**caractérisé en ce que** ledit dispositif comprend en outre des moyens de mesure (8) pour mesurer la distance de l'oeuf (9) à mirer par rapport aux moyens de réception (104), le traitement informatique du flux lumineux reçu par lesdits moyens de réception étant effectué en fonction de la distance mesurée.

2. Dispositif de mirage selon la revendication 1, **caractérisé en ce que** ladite source laser (3, 103) est disposée en-dessous des oeufs (9), le faisceau optique formant un angle non nul par rapport à la verticale.

3. Dispositif de mirage selon la revendication 2, **caractérisé en ce que** ladite source laser (3, 103) est disposée en-dessous d'une plaque protectrice (6, 106).

4. Dispositif de mirage selon la revendication 1, **caractérisé en ce que** ladite source laser (203) est disposée au-dessus des oeufs, le faisceau cohérent (231) formant un angle non nul par rapport à la verticale.

5. Dispositif de mirage selon l'une des revendications 1 a 4, **caractérisé en ce que** les moyens de réception (4, 104, 204) sont disposés du côté du plan médian vertical de l'oeuf opposé à ladite source laser, au-dessus des oeufs à mirer.

6. Dispositif de mirage selon l'une des revendications 1 a 5, **caractérisé en ce que** ladite source laser (3, 103, 203) est équipée de moyens de focalisation pour former un faisceau optique concentré.

7. Dispositif de mirage selon l'une des revendications 1 a 6, **caractérisé en ce que** ladite source laser (3, 103, 203) est constituée d'une diode laser équipée d'un collimateur laser.

8. Dispositif de mirage selon l'une des revendications 1 a 7, **caractérisé en ce que** ladite source laser est une source laser infrarouge.

9. Dispositif de mirage selon l'une des revendications 1 a 8, **caractérisé en ce que** lesdits moyens de réception comprennent pour chaque oeufs au moins une photodiode (4, 104, 204), ayant une surface de réception d'au moins 1 mm², équipée de moyens de focalisation du flux lumineux transmis à travers l'oeuf.

10. Dispositif de mirage selon l'une des revendications 1 a 9, **caractérisé en ce qu'**il comprend des moyens de convoyage (1) pour transporter des plateaux (2) munis d'alveoles (21) dans lesquelles des oeufs (9) a mirer sont disposés, lesdits moyens d'émission comprenant au moins une rangée de sources laser cohérentes disposées transversalement à la direction d'avancement (F1) des plateaux pour émettre au moins un faisceau optique (31, 20 131, 231) vers chacun des oeufs d'une même rangée d'un plateau, et au moins une rangée de moyens de réception pour recevoir les flux lumineux provenant des oeufs d'une rangée d'un plateau.

11. Dispositif de mirage selon l'une des revendications 1 a 10, **caractérisé en ce qu'**il comprend en outre des moyens de synchronisation pour synchroniser les mesures de transparence effectuées par les moyens de réception et les impulsions de la source laser sur la fréquence de l'énergie des ondes lumineuses ambiantes, au voisinage du sommet S des périodes T de l'énergie des ondes lumineuses ambiantes.

## Patentansprüche

1. Vorrichtung zum automatischen Durchleuchten von Eiern, die ermöglicht, die befruchteten Eier von den unbefruchteten Eiern zu unterscheiden, umfassend Mittel zum Aussenden eines Lichtstroms in Richtung eines zu durchleuchtenden Eis, Mittel zum Empfangen des Lichtstroms, der durch das Ei hindurchgeht, und Mittel zur Datenverarbeitung des Lichtstroms, der von den Mitteln zum Empfangen empfangen wird, um den befruchteten oder unbefruchteten Zustand des Eis zu bestimmen, wobei die Mittel zum Aussenden (3, 103, 203) für jedes zu durchleuchtende Ei mindestens eine kohärenten Laserquelle aufweisen, die einen kohärenten optischen Strahl (31, 131, 231) in Richtung des Eis (9) bildet, **dadurch gekennzeichnet, dass** die Vorrichtung ferner Messmittel (8) zum Messen des Abstands des zu durchleuchtenden Eis (9) zu den Mitteln zum Empfangen (104) aufweist, wobei die Datenverarbeitung des Lichtstrahls, der von den Mitteln zum Empfangen empfangen wird, in Abhängigkeit von dem gemessenen Abstand durchgeführt wird.

2. Vorrichtung zum Durchleuchten von Eiern nach Anspruch 1, **dadurch gekennzeichnet, dass** die Laserquelle (3, 103) unter den Eiern (9) angeordnet ist, wobei der optische Strahl einen von Null verschiedenen Winkel bezüglich der Vertikale bildet.

3. Vorrichtung zum Durchleuchten von Eiern nach Anspruch 2, **dadurch gekennzeichnet, dass** die Laserquelle (3, 103) unter einer Schutzplatte (6, 106) angeordnet ist.

4. Vorrichtung zum Durchleuchten von Eiern nach Anspruch 1, **dadurch gekennzeichnet, dass** die Laserquelle (203) über den Eiern angeordnet ist, wobei der kohärente Strahl (231) einen von Null verschiedenen Winkel bezüglich der Vertikale bildet.

5. Vorrichtung zum Durchleuchten von Eiern nach einem der Ansprüche 1 bis 4, **dadurch gekennzeichnet, dass** die Mittel zum Empfangen (4, 104, 204) auf der Seite der vertikalen Mittelebene des Eis gegenüberliegend von der Laserquelle über den zu durchleuchtenden Eiern angeordnet sind.

6. Vorrichtung zum Durchleuchten von Eiern nach einem der Ansprüche 1 bis 5, **dadurch gekennzeichnet, dass** die Laserquelle (3, 103, 203) mit Fokussiermitteln ausgestattet ist, um einen konzentrierten optischen Strahl zu bilden.

7. Vorrichtung zum Durchleuchten von Eiern nach einem der Ansprüche 1 bis 6, **dadurch gekennzeichnet, dass** die Laserquelle (3, 103, 203) aus einer Laserdiode gebildet ist, die mit einem Laser-Kollimator ausgestattet ist.

8. Vorrichtung zum Durchleuchten von Eiern nach einem der Ansprüche 1 bis 7, **dadurch gekennzeichnet, dass** die Laserquelle eine Infrarotlaserquelle ist.

9. Vorrichtung zum Durchleuchten von Eiern nach einem der Ansprüche 1 bis 8, **dadurch gekennzeichnet, dass** die Mittel zum Empfangen für jedes Ei mindestens eine Photodiode (4, 104, 204) aufweisen, die eine Empfängerfläche von mindestens 1 mm² aufweist, die mit Fokussiermitteln des Lichtstroms ausgestattet ist, der durch das Ei gesendet wird.

10. Vorrichtung zum Durchleuchten von Eiern nach einem der Ansprüche 1 bis 9, **dadurch gekennzeichnet, dass** sie Fördermittel (1) zum Transportieren von Einsätzen (2), die mit wabenartigen Vertiefungen (21) ausgestattet sind, in denen die zu durchleuchtenden Eier (9) angeordnet sind, wobei die Mittel zum Aussenden mindestens eine Reihe von kohärenten Laserquellen umfassen, die quer zu der Vorschubrichtung (F1) der Einsätze angeordnet sind, um mindestens einen optischen Strahl (31, 20, 131, 231) zu jedem der Eier von einer gleichen Reihe eines Einsatzes zu senden, und mindestens eine Reihe von Mitteln zum Empfangen aufweist, um die Lichtflüsse zu empfangen, die von den Eiern von einer Reihe von einem Einsatz kommen.

11. Vorrichtung zum Durchleuchten von Eiern nach einem der Ansprüche 1 bis 10, **dadurch gekennzeichnet, dass** sie ferner Synchronisationsmittel aufweist, um die Transparenzmessungen, die durch die Mittel zum Empfangen durchgeführt werden, und die Impulse der Laserquelle auf der Frequenz der Energie der umgebenden Lichtwellen in der Nähe des Scheitelpunkts S der Perioden T der Energie der umgebenden Lichtwellen zu synchronisieren.

## Claims

1. Automatic egg examining device for differentiating between fertilized and unfertilized eggs, comprising means for emitting a light beam in the direction of an egg to be examined, means for receiving the light beam passing though the egg, and means for processing data regarding the light beam received by said receiving means in order to determine the fertilized or unfertilized state of the egg, said emission means (3, 103, 203) comprising, for each egg to be examined, at least one coherent laser source forming a coherent optical beam (31, 131, 231) in the direction of the egg (9), **characterized in that** the examining device further comprises measuring means (8) for measuring the distance of the egg (9) to be examined relative to the receiving means (104), the data regarding the light beam received by said receiving means being processed as a function of the distance measured.

2. Examining device according to claim 1, **characterised in that** said laser source (3, 103) is arranged below the eggs (9), the optical beam forming a non-null angle with respect to the vertical.

3. Examining device according to claim 2, **characterised in that** said laser source (3, 103) is arranged below a protective plate (6, 106).

4. Examining device according to claim 1, **characterised in that** said laser source (203) is arranged above the eggs, the coherent beam (231) forming a non-null angle with respect to the vertical.

5. Examining device according to any of claims 1 to 4, **characterised in that** the receiving means (4, 104, 204) are arranged to the side of the vertical median plane of the egg opposite said light source, above the eggs to be examined.

6. Examining device according to any of claims 1 to 5, **characterised in that** said laser source (3, 103, 203) is provided with focusing means for forming a concentrated optical beam.

7. Examining device according to any of claims 1 to 6, **characterised in that** said laser source (3, 103, 203) is constituted by a diode laser provided with a laser collimator.

8. Examining device according to any of claims 1 to 7, **characterised in that** said laser source is an infrared laser source.

9. Examining device according to any of claims 1 to 8, **characterised in that** said receiving means comprise, for each egg, at least one photodiode (4, 104, 204), having a receiving surface area of at least 1 mm2, provided with means for focusing the light beam passing through the egg.

10. Examining device according to any of claims 1 to 9, **characterised in that** it comprises conveying means (1) for transporting trays (2) provided with wells (21) in which the eggs (9) to be examined are arranged, said emission means comprising at least one row of coherent laser sources arranged transversely to the direction of movement (F1) of the trays so as to emit at least one optical beam (31, 131, 231) towards each of the eggs in a single row of a tray, and at least one row of receiving means for receiving the light beams coming from the eggs in a row of a tray.

11. Examining device according to any of claims 1 to 10, **characterised in that** it further comprises synchronisation means for synchronising the transparency measurements taken by the receiving means and the pulses of the laser source on the energy frequency of the ambient light waves in the vicinity of the peak S of the time periods T of the energy of the ambient light waves.
